# EUROPÄISCHE PATENTSCHRIFT

(11) **EP 1 696 971 B1**
(45) Veröffentlichungstag und Bekanntmachung des Hinweises auf die Patenterteilung: **11.04.2012**
(21) Anmeldenummer: 04803360.9
(22) Anmeldetag: 30.11.2004
(51) Int. Cl.: A61L 15/28, A61L 15/32, A61L 15/38, A61L 15/40

(54) **THERAPEUTISCH AKTIVE WUNDAUFLAGEN, DEREN HERSTELLUNG UND DEREN VERWENDUNG**
THERAPEUTICALLY ACTIVE WOUND DRESSINGS, PRODUCTION THEREOF, AND USE OF THE SAME
PANSEMENTS POUR PLAIES A ACTION THERAPEUTIQUE, LEUR PRODUCTION ET LEUR UTILISATION

(30) Priorität: 26.12.2003 RU 2003138256
(43) Veröffentlichungstag der Anmeldung: 06.09.2006
(73) Patentinhaber: SanguiBioTech GmbH, 58455 Witten (DE); Teslenko, Alexander, 58095 Hagen (DE)
(72) Erfinder: TESLENKO, Alexander, 58095 Hagen (DE); NIKONOW, Boris Alekseevich, S-Petersburg, 196105 (RU); ANTONOW, Sergej Fedorovich, Nikolskoye, 187026 (RU)
(74) Vertreter: Müller, Claudia
(86) Internationale Anmeldenummer: PCT/EP2004/013575
(87) Internationale Veröffentlichungsnummer: WO 2005/063311

(56) Entgegenhaltungen:
- EP-A- 0 049 177
- EP-A- 0 560 014
- EP-A- 1 118 705
- EP-A- 1 120 428
- EP-A- 1 245 239
- WO-A-02/051457
- WO-A-2004/080500
- DE-A1- 19 712 699

## Beschreibung

### Gegenstand der Erfindung

Die Erfindung betrifft therapeutisch aktive Wundauflagen auf Polysaccharid, insbesondere, Chitosan, und Eiweiß, insbesondere Kollagen /Gelatine Basis, mit verbesserten Eigenschaften, deren Herstellung, insbesondere unter Anwendung von Polycarbonsäuren sowie vorzugsweise polyfunktionellen Aminosäuren, und gemeinsamer Polymerdialyse, sowie deren Verwendung, vor allem im medizinischen Bereich.

### Stand der Technik

Die Zahl der Patienten mit chronischen schlecht heilenden Wunden steigt trotz enormer Fortschritte in der Medizin mit zunehmender Tendenz. In der lokalen Wundbehandlung sind bereits verschiedene chemisch-pharmazeutische Präparate in unterschiedlichen Darreichungsformen als Salben, Gele, Pflaster, Filme, Puder und andere bekannt. Wie allein die Vielfältigkeit von Wundheilendem Präparaten deutlich zeigt, gibt es heutzutage kein universelles Präparat. Lokale Wundbehandlung bedeutet, schädliche Faktoren von einer Wunde fern zu halten und heilungsfördemde Mechanismen zu unterstützen. Für die Entwicklung eines solchen medizinischen Produktes sind die folgenden Parameter von Bedeutung: antibakterielle Wirkung, wundheilende Wirkung und nicht zuletzt eine gute Handhabung, z. B. leichte Ablösbarkeit von der Wunde usw.

Unabhängig von der Wundart und vom Ausmaß des Gewebeverlustes verläuft jeder Wundheilungsprozess in drei sich überlappenden und nicht von einander zu trennenden Phasen ab: Entzündung, Proliferation und Modulation. Das Immunsystem hat bei der Wundheilung eine zentrale Bedeutung. Immunkompetente Zellen integrieren sich innerhalb der mesenchymalen Zellen des neuen Granulationsgewebes und steuern ein komplexes Netzwerk aus Zellen der extrazellulären Matrix und zellulären Mediatoren.

Die biologisch-aktiven und pharmakologischen Stoffe sind unverzichtbare Anteile bei der effizienten Wundbehandlung. Wunden, die sich in der ersten Wundheilungsphase befinden, sind schmerzhaft und schwer erträglich für einen Patienten. Die für Schmerzen verantwortlichen Entzündungsprozesse sind von einer ganzen Reihe Faktoren, u.a. aktiven Sauerstoffradikalen sog. ROS (Hydroxyl-Radikale und Superoxid-Anionen) abhängig. Deswegen geht man davon aus, dass, wenn man das Niveau der Radikalkonzentration in der Wunde herabsetzt, gleichfalls der Schmerz in seiner Intensität herabgesetzt wird. Es ist bekannt, dass Chitosan freie Radikale fangen kann (Park P.J., et al. J. Agric. Food Chem. 2003, 51 (16): 4624-7; Je JY, et al. Food Chem. Toxicol. 2004, 42 (3): 381-7). Die Effizienz ist jedoch nicht ausreichend. Eine erhebliche Verstärkung des Neutralisationsprozesses von ROS erreicht man durch Zugabe von Superoxiddismutase (SOD) und Katalase in die Wundauflage. Die Dismutase zerlegt das Superoxid-Anion und die Katalase Wasserstoffperoxid: Beide gehören zu den schädlichen Sauerstoff reaktiven Stoffen. Laut Stand der Technik gibt es bisher keine SOD und Katalase enthaltenden Wundauflagen.

Ferner ist bekannt, dass Retinoide bzw. Vitamin A bei der Epitheliasierung und Kontraktion der Wunde eine wichtige Rolle spielen. Die Anwendung von Retinoiden bei der Behandlung verschiedener Hauterkrankungen ist bekannt. Es wurde nachgewiesen, dass Retinoide bei der Biosynthese und Katabolismus von Kollagen eine wichtige Rolle spielen. Sie verhindern die Expression von Kollagenase und erhöhen die Expression von Metalloprotease-Inhibitoren in menschlichen Fibroblasten (Bizot-Foulon V. et al. Cell Biol. Int. 1995. 19 (2): 129-35). Gleichzeitig ist bekannt, dass eine hohe Retinoiddosis zu unerwünschten Effekten auf der Haut führt (Erythema-Disquamation und Dermatiden).

Ein Einschließen von Retinoiden in Liposomen kann die Bioverträglichkeit deutlich verstärken und gleichzeitig eine schnelle Inaktivierung verhindern (Bizot-Foulon V. et al. Int. J. Cosmetic Sci. 1998, 20 (2): 343-354). Wundauflagen mit derartiger Substanzen sind jedoch bisher nicht beschrieben.

Chronische Wunden wie diabetischer Fuß, Decubitus oder verursacht durch venöse und arterielle Insuffizienz befinden sich unter Sauerstoffmangel (Hypoxie). Die Höhe des Sauerstoffpartialdruckes in der Wunde ist ein entscheidender Parameter für eine Amputation des Organes. Es ist bekannt, dass die Aminosäure Arginin das Risiko einer Vasculopathie verhindern kann (US 5359007, 2002). An experimentell erzeugten diabetischen Wunden an Tieren wurde gezeigt, dass Arginin die Wundheilung verbessert bzw. die Kollagenbiosyntheserate erhöht (Shi H.P., et al. Wound Repair Regener. 2003. 11(3): 198-203).

So weist z. B. die Aminosäure Taurin antioxidative Aktivität auf (Franconi F. et al. Neurochem. Res. 2004, 29(1): 143-150), stimuliert die Zellproliferation und die Kollagenbiosynthese. Taurin ist in der Lage, die Monochloramine, die durch Neutralisierung von Hypochloriden entstanden sind, abzufangen und dadurch den Wundheilungsprozess zu verbessern (Kato S., et al. Aliment. Pharmacol. Therap., 2002, 16 (2): 35-43). Somit scheinen Arginin, Taurin und andere polyfunktionelle Aminosäuren ohne Zweifel von großer Bedeutung bei der Entwicklung von pharmakologisch aktiven Wundauflagen. Außerdem spielen sie als Bausteine für die Biosynthese von Kollagen und anderen Eiweißen und Glycoproteinen in der Wunde eine wichtige Rolle.

Wundauflagen sind unverzichtbar in der modernen Wundversorgung Eine effiziente Wundauflage, als provisorische Gewebematrix, sollte eine ganze Reihe von Funktionen erfüllen: Neben dem Schutz vor negativen Umwelteinflüssen (z. B. bakterielle Infektionen) als Abdeckungsmaterial, gutem Wasser- und Gasaustausch, guter Sorptionskapazität für Wasser und Toxine (z. B. Endotoxine oder Entzündungsmediatoren) sollte außerdem eine Wundauflage möglichst als Gerüst (als Ersatz für eine natürliche extrazelluläre Matrix) für das neue Zellwachstum dienen und dieses zumindest positiv beeinflussen aufgrund von eigener biologischer Aktivität oder anwesenden biologischen Wirkstoffen. Ferner sollte sie als Depot für die bereits oben erwähnten therapeutischen Präparate dienen können.

Besonderes Interesse finden hydrophile Wundauflagen als poröse Materialien (US 4 572 906, 1986; US 4 570 696,1986; US 4 659 700, 1987; US 4 956 350, 1990; US 5 169 630,1992; US 5 324 508, 1994; US 5 871 985, 1999; US 6 509 039, 2003; US 6 608 040, 2003, RU 2007180, 1994; RU 2028158, 1996, RU 2193895,2002) und insbesondere als Schwämme und Membranen.

Problematisch ist bei diesen Materialien (Membranen und Folien), dass nur kleine Poren vorhanden sind, die für die Migration von Zellen (Fibroblasten, Keratinocyten und andere) zu klein sind und letztendlich nicht das dreidimensionale Wachstum von Granulationsgewebe ermöglichen. Außerdem ist das Material aufgrund der niedrigen Porosität nicht in der Lage, große Mengen von Exsudat aus der Wunde zu adsorbieren.

Poröse Schwämme haben im Gegensatz dazu bessere Eigenschaften (US 5 116 824,1992; US 2002161440, 2002; DE 101 17234 A1, 2002).

Körpereigenes Kollagen spielt insbesondere bei einem Wundheilungsprozess eine wichtige Rolle. Nach Spaltung durch Kollagenase können freigesetzte Kollagenabbauprodukte sowohl die Migration als auch die Aktivierung von Entzündungszellen z. B. Makrophagen verursachen und beeinflussen, somit den Heilungsprozess schon in einem frühen Stadium. Aus diesem Grunde sind die Wundauflagen und hämostatischen Schwämme auf Kollagen- und Gelatinebasis weit verbreitet: Drop Collagen® (Master Aid), AngioSeal® (Wright Medical Biomaterial Products), Nobakoll® (NOBA), PolyPly® (Royce Medical), Matrix Collagene® (Collagen matrix, Inc.), Suprasorb C® (Lohmann & Rauscher GmbH). Die Hauptfunktion bleibt jedoch nur die passive physikalische Eigenschaft, nämlich die Wundabdeckung und Adsorption von Exsudat.

Daher wurden auch Wundauflagen mit therapeutisch wirksamen Substanzen entwickelt. Hier sind solche auf Basis von Kollagen B (SU 561564, 1965), Chitin, Gelatine und Formaldehyd (CN1097980, 1995), Gelatine-Formaldehyd mit Antibiotikum (RU 2033149, 1995), Zellulose mit Chitosan (WO 02/052028), Kollagen und Chitosan (PCT 8504413,1986), Kollagen und Chitosan mit Antibiotikum (RU 96124444,1998) bekannt. Der Nachteil solcher Wundauflagen ist aber die geringe Effizienz bei der Wundheilung, die durch ein Verkleben der Wunde und bei Kollagenschwämmen auf Grund nicht ausreichender Sauerstoffversorgung der Wunde hervorgerufen wird.

Es ist aber auch bekannt, dass Kollagen mit nativer fibrillärer Struktur die beste Bioverträglichkeit beim Wundheilungsprozess aufweist (US 4378017, 1983). Die quartären Biopolymerstrukturen einschließlich Proteinen werden durch eine Hydrathülle an polaren Gruppen von Polymeren stabilisiert. Wenn eine solche Wechselwirkung zwischen Wasser und polaren Gruppen von Biopolymeren nicht zustande kommt, wird bei Entfernung von Wasser (Trocknungsprozess) die Struktur des Biopolymers beeinträchtigt. Manche niedrig molekularen Stoffe sog. kosmotropische Agentien sind in der Lage, die vorhandene Hydrathülle zu stabilisieren und dadurch die makromolekularen Strukturen während des Trocknungsprozesses und danach zu erhalten. Nach solchen Stabilisatoren und deren Anwendung wird experimentell gesucht (Crowe L.M. et al. Interaction of sugar with membranes: Biochim. et Biophys. Acta, 1988, 947, 367-384. Carpenter J. F. et al. The mechanism of cryoprotection of proteins by solutes ; Cryobiology 1988, 25, 244-255). Traditionell werden bei der Gefriertrocknung von Eiweißen Mono-und Oligosaccharide verwendet. Es zeigte sich, dass diese gerade nicht geeignet sind bei der Herstellung erfindungsgemäßer Schwämme.

Die Effizienz derartiger Wundauflagen und Membranen auf Kollagen- und GelatineBasis wurde erheblich verbessert durch Beimischung bioverträglicher Polymere, z. B.: Oxy-Cellulose: Promogran® (Johnson & Johnson), Alginsäure: Fibracol® (Johnson & Johnson) oder Mucopolysaccharide: Catrix® (Lescander, Inc.) und andere.

Hierbei ist nicht toxisches und bioverträgliches Chitosan besonders interessant wegen seiner besonderen Eigenschaften: das kationische Polysaccharid Chitosan bildet mit anionischen Molekülen und Polymeren ionische Komplexe. Das findet seinen Einsatz u.a. bei der Immobilisierung einer Reihe von therapeutischen und biologisch aktiven Substanzen, z. B. die Immobilisierung von Eiweißen, Mikroorganismen oder für die Bindung bakterieller Endotoxine (Davidova VN et al., Biochemistry (SU), 2000, 65 (9), 1082-90).

Das heterogene Polysaccharid Chitosan besteht aus N-Acetyl-D-Glucosamin und D-Glucosamin und hat chemische Ähnlichkeit mit Glucosaminoglycanen der Haut. Daraus resultieren eine Reihe interessanter biologischer Eigenschaften von Chitosan (z. B. Makrophagenaktivierung, Immunostimulierung und andere, in Khor E. "Chitin. Fulfilling a Biomaterials Promise. Elsevier, Amsterdam, 2001).

In zahlreichen Untersuchungen wurde nachgewiesen, dass nicht Chitin/Chitosan, sondern deren Spaltprodukte, nämlich Chito-Oligosaccharide (COS), biologische Aktivität zeigen /Muzzarelli R.A.A.(ed) Chitosan per os, from dietary supplement to drug carrier AtecEdizioni. 2000./. Nach neuestem Kenntnisstand spielt im Rahmen der menschlichen Physiologie die enzymatische Aktivität von hydrolytischen Enzymen, wie Chitinase, Lysozym oder Hexoaminidase, eine entscheidende Rolle. Auch Makrophagen sind in der Lage, eine große Menge von diesen Enzymen im menschlichen Körper zu produzieren /Muzzarelli R.A.A. Human enzymatic activities related to the therapeutic administration of chitin derivates. CMLS. Cellular Molec. Life Sci. 1997, 53: 131-140.

Die einzigartigen Eigenschaften von Chitosan im Zusammenwirken mit anderen Polymeren z. B. Gelatine oder Kollagen sind wie erwähnt von großer Bedeutung bei der Entwicklung pharmazeutisch aktiver Wundauflagen und Bioprothesen. In der Fachwelt sind neben den bereits genannten noch die folgenden Materialien bekannt: Chitosan/Kollagen- und Chitosan/Gelatine-Schwämme (US 4659700, 1987; US 5166187, 1992; 5116824, 1992; US 5836970, 1998; US 5871985, 1999; US 2002161440, 2002; US 6565878, 2003). In diesem Zusammenhang sind vor allem die folgenden Patente über Wundauflagen auf Chitosan-Kollagen-Basis zu nennen: US 5116824, 1992; US 5166187, 1992, US 5836970, 1998; US 2002161440, 2002; US 6565878,2003; RU 8608 B, 1998. In den zitierten Patenten wird Chitosan in Essigsäure gelöst, die im weiteren Verlauf neutralisiert wird. Ein erheblicher Nachteil dieses Prozesses besteht darin, dass heterogene Lösungen gebildet werden, aus denen sich nach Wasserentfemung Schwämme mit unerwünschter Morphologie bilden, nämlich mit niedriger Porosität, mit nicht optimaler Porenform und Porengröße für Zellwachstum. Das beeinträchtigt jedoch die Effizienz der praktischen Anwendung. So beträgt die Wasseraufnahme für solche Materialien nur 1000 bis 2000 % des eigenen Gewichtes. Ein Nachteil der in den oben zitierten Patenten beschriebenen Technologien besteht darin, dass ein hoher Anteil Ausschuss produziert wird (bis 70 %).

So wird durch Lyophilisierung die Kontraktion von Schwämmen beobachtet, verbunden mit einem Verlust an porösen Strukturen. Ferner erhält man eine nicht ebene Oberfläche für die Wundauflagen, so dass sie nur sehr schlecht die Wunde optimal abdecken. Es wurde auch festgestellt, dass die Essigsäurereste in der Wundauflage die Wunde reizen können. Um den Überschuss von Essigsäure zu entfernen, wurden daher die Polymerlösungen bis sechs Tage intensiv dialysiert. Aber selbst nach dieser Behandlung waren immer noch Reste von Essigsäure nachweisbar.

In der DE 197 12 699 A1 werden Gewirke, Gewebe, Vliese und Multi-Watte-Kompressen beschrieben, welche wundversorgungsaktive Stoffe, Polysaccharide wie Chitosane, sowie Eiweißlysate in einer Menge von 2% aufweisen. Derartige Produkte sind nicht vernetzt und bedürfen daher eines Trägers.

### Aufgabe vorliegender Erfindung

Ausgehend von diesen Erkenntnissen ist es die Aufgabe der vorliegenden Erfindung, neue pharmakologisch aktive Wundauflagen auf Polysaccharid und Eiweiß - Basis zu entwickeln, welche verbesserte therapeutische Eigenschaften aufweisen. Insbesondere sollen wirksame Substanzen wie z. B., SOD, andere Enzyme und Cytokine, polyfunktionelle Aminosäuren, oder auch beispielsweise liposomal verkapselter Retinol, enthalten und Hilfsstoffe inkorporierbar sein. Die Stabilität von Eiweißen und die Porosität von Wundauflagen soll so beschaffen sein, dass ein verbessertes Wasseraufnahmevermögen und eine hohe Effizienz erreicht wird, also die vorstehend genannten Nachteile überwunden werden. Die Wundauflage soll ferner auf verschiedene Wundtypen und Heilungsphasen wirken, also insofern eine gewisse Universalität aufweisen.

### Lösung der Aufgabe

Erfindungsgemäß wird in einem neuen Verfahren eine Wundauflage hergestellt auf Basis eines strukturellen Polysaccharids, insbesondere Chitosan, und Strukturellen Eiweißen, insbesondere Kollagen und/oder Gelatine, welche gekennzeichnet ist durch einen Anteil an Eiweißen, ausgewählt aus Kollagen, Gelatine, Derivaten oder Mischungen hiervon, Chitosan oder Derivaten hiervon, sowie Polycarbonsäuren/ Carbonsäuren und polyfunktionelle Aminosäuren/Aminosäuren, sowie ggf. einen Anteil an wirksamen Substanzen und welche als Rest Hilfsstoffe und/oder Zusatzstoffe enthält. Die Wundauflage ist ferner vernetzt. Überraschenderweise können hiermit, und vor allem bei Verzicht auf die Monokarbonsäure Essigsäure, die oben beschriebenen Nachteile überwunden werden, wie nachfolgend erläutert.

Dabei dokumentieren die
Beispiele 1 bis 9 die Herstellung und Eigenschaften erfindungsgemäßer Auflagen, auch im Vergleich mit bekannten Produkten.
Beispiele 10 und 11 belegen die verbesserte Wirksamkeit erfindungsgemäßer Auflagen.
Abbildung 1 zeigt die Bildung von Fibrillen einer Kollagen-Chitosan Mischung hergestellt nach Stand der Technik (RU 8608, Essigsäure);
Abbildung 2 zeigt die verbesserte Bildung von Fibrillen einer Kollagen-Chitosan Mischung hergestellt gemäß der Erfindung mit Polycarbonsäure.
Abbildung 3 zeigt die zusätzliche Verbesserung der Wundauflagen durch den Einfluss von Hilfsstoffen.

### Kurze Erläuterung der Zeichnungen

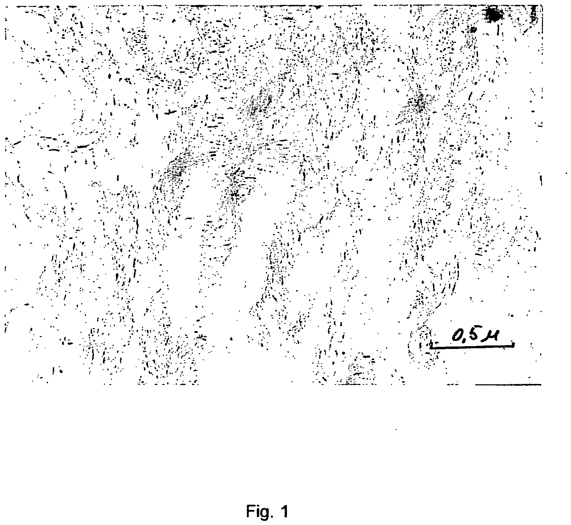

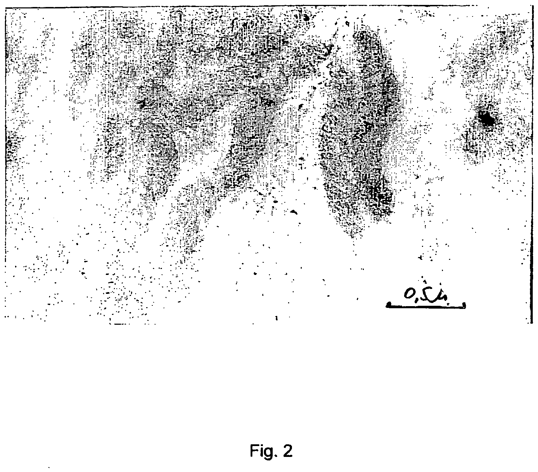

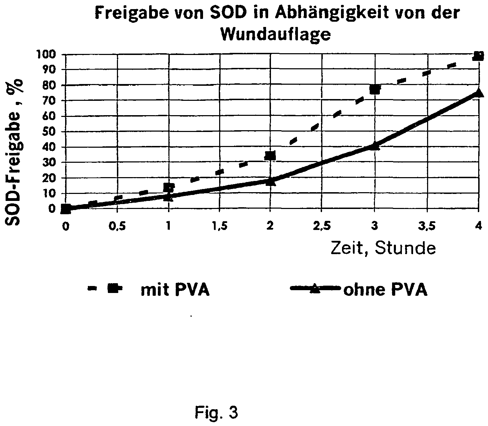

In Fig. 1 und 2 ist die Bildung von Fibrillen einer Kollagen-Chitosan Mischung dargestellt, und zwar einmal gemäß RU 8608 (Essigsäure), Fig. 1 und einmal mit Polycarbonsäure (Erfindung) Fig. 2. In Fig. 3 ist die prolongierte Wirkung von Superoxiddismutase bei einer Wundauflage ohne und einer mit Polyvinylalkohol (PVA) gezeigt.

### Nähere Erläuterung der Erfindung

Die Wundauflage verfügt insbesondere über einen Anteil von 19 bis 56% Strukturelle Eiweiße, ausgewählt aus Kollagen, Gelatine, Derivate oder Mischungen hiervon, 18 bis 58% strukturelle Polysaccharide, ausgewählt aus Chitosan oder Derivaten hiervon, 0,5 bis 10 % Polycarbonsäuren/Carbonsäuren, 0,1 bis 15 % polyfunktionelle Aminosäuren/Aminosäuren, 0 bis 10% wirksame Substanzen und 0 bis 30% Zusatz- und oder Hilfsstoffe neben 0,1 bis 5 % Vernetzer. Sie hat ferner vor allem ein Wasseraufnahmevermögen von mehr als 2000%, vor allem 2500 bis 10000%, vorzugsweise 3000 bis 7000%. Sie hat eine poröse (Schwamm) Struktur. In besonders vorteilhaften Ausgestaltungen weist sie ein spezifisches Gewicht von 0,01 bis 0,06 g/cm³ auf.

Ganz besonders bevorzugt ist hierbei Chitosan mit einem mittleren Molekulargewicht von größer 200 bis 500 KD, Kollagen Typ I und III und Gelatine vom Typ A oder B.

Unter Polycarbonsäuren werden gemäß vorliegender Erfindung solche Carbonsäuren verstanden, die zusätzlich zu einer Carboxylgruppe eine oder mehrere funktionelle Gruppen (Hydroxy-, Carboxy-, Amino-u.a.) enthalten.

Als Polycarbonsäuren eignen sich vor allem Milchsäure, Äpfelsäure, Bernsteinsäure, Pyrrolidoncarbonsäure, Malonsäure, Fumarsäure, Ascorbinsäure, Glutaminsäure, Salicylsäure u.a. oder Mischungen hiervor. Besonders bevorzugt ist Milchsäure, Bernsteinsäure und andere aus den Zitratzyklusses.

Als polyfunktionelle Aminosäuren kommen bevorzugt Arginin, Methionin, Prolin, Glutaminsäure, Alanin, Taurin, Glycin Cystein, N-Acetylcystein oder Mischungen hiervon in Frage.

Als wirksame Substanz wird insbesondere Superoxiddismutase und/oder Katalase von verschiedenem Ursprung, vor allem in einer Konzentration von 0,001 bis 1,0 % zur Polymerbasis gewählt. Alternativ oder zusätzlich kann als pharmakologisch aktiver Stoff ß-Carotin von verschiedenem Ursprung, insbesondere in liposomaler Form, enthalten sein, wobei ß-Carotin bevorzugt in einer Konzentration von 0,001 bis 0,5 % zur Polymerbasis eingesetzt wird.

Als Zusatzstoffe sind vor allem antibakterielle Stoffe wie insbesondere Chlorhexidin, Polysept, Polihexanid bzw. geeignete Derivate hiervon geeignet. Diese können bevorzugt in einer Konzentration von 0,01 bis 0,6 % zur Polymerbasis eingesetzt werden. Hilfsstoffe sind in einer bevorzugten Ausführungsform ausgewählt aus Plastifikatoren wie Glycerin, oder hochmolekularen Stoffen, die die Adhäsion zur Wundoberfläche gewährleisten oder Stoffen, die die Ausscheidung von pharmazeutisch aktiven Stoffen beeinflussen, oder Mischungen hiervon. Besonders geeignet sind Mengen an Hilfsstoffen von 10 - 30 %. In einer vorteilhaften Ausführungsform werden als Hilfsstoffe Glycerin und andere Polyole, insbesondere Polyvinylalkohol und Polyvinypyrrolidon verwendet.

Als Vernetzer wird insbesondere ein bifunktioneller Vernetzer, bevorzugt in einer Menge von 0,1 bis 5%, vor allem Glutardialdehyd, gewählt.

Die erfindungsgemäßen Wundauflagen werden nach einem neuen Verfahren hergestellt, mit welchem die Ausbeute an Produkten (Schwämmen) geeigneter Struktur erheblich gesteigert werden kann.

Der Herstellungsprozess erfolgt in folgender Weise:
1. Anstelle von Essigsäure werden Polycarbonsäuren, einschließlich der Säuren, die zum Citratzyklus gehören d. h. mit biologischer Aktivität, verwendet (Tab.1 bis 4).
   Insbesondere wird es weiterhin bevorzugt, dass
2. Anstelle einer Neutralisation ein Dialyseprozess vorgenommen wird, der zur Bildung von optimalen Eiweißstrukturen (z. B. Kollagenfasern) führt.
3. Des Weiteren können überraschenderweise polyfunktionelle Aminosäuren eingesetzt werden. Diese führen zum einen zu einer Stabilisierung von quartärneren Strukturen von Eiweißverbindungen. Sie spielen eine wichtige Rolle als Kryoprotektoren und Porenbildner. Zum anderen zeigen sie eigene biologische Aktivität und beschleunigen damit den Wundheilungsprozess. Dies ist im Gegensatz zur Lehre gemäß Stand der Technik, worin Mono- und Disaccharide als übliche Stabilisatoren vorgeschlagen wurden. Es zeigte sich, dass diese gerade nicht geeignet sind bei der Herstellung erfindungsgemäßer Schwämme. Es kann also überraschenderweise auf diese Substanzen verzichtet werden, wobei auch deren unerwünschte Wirkung auf die Wunde vermieden wird.
4. Des Weiteren können überraschenderweise SOD, andere Enzyme, ß-Karotin, andere Provitamine und andere pharmakologisch aktive Stoffe eingesetzt werden.
5. Des Weiteren können überraschenderweise Antiseptika und Hilfsstoffe eingesetzt werden.

Der erfindungsgemäße Herstellungsprozess besteht darin, dass das strukturelle Polysaccharid, insbesondere Chitosan, und das strukturelle Einweiß, insbesondere Kollagen, Gelatine oder Kollagen-Gelatine-Mischungen, jeweils getrennt mit Polycarbonsäuren, welche gleich oder verschieden sein können, in Wasser gelöst und danach zusammen gemischt und dialysiert werden. Anschließend werden bevorzugt Kryoprotektoren und Strukturbildner, ausgewählt aus polyfunktionellen Aminosäuren, und Zusatzstoffe eingebracht und das daraus entstehende Gel gefriergetrocknet.

Die Polycarbonsäuren werden bevorzugt in einem Verhältnis von 1 : 4 bis 2 : 1 auf Trockengewichtsbasis und die Aminosäuren in einer Konzentration von 0,1 - 15 % eingesetzt (s. Tab.3).

Als Polycarbonsäuren mit dem Ziel der optimalen Bioverträglichkeit werden vor allem die folgenden Säuren eingesetzt: Milchsäure, Malonsäure, Bernsteinsäure, Fumarsäure oder deren Mischungen als Komponenten des Citratzyklusses verwendet. Die Mischungen der Polysaccharid insbesondere Chitosan- und Eiweiß, insbesondere Kollagen- (Gelatine-) Lösungen werden vermischt und insbesondere mindestens 12 Stunden ausgereift und anschließend dialysiert.

Der Dialyseprozess wird abgebrochen bei Erreichen eines pH-Wertes von 5,2 - 6.5 in Abhängigkeit von der Zusammensetzung. Der Dialyseprozess hat einen großen Einfluss auf die Verfügbarkeit der pharmazeutisch aktiven Substanzen (z.B. SOD) aus der fertigen Wundauflage in die Wunde (s. Tab. 5.). So ist es bevorzugt, wenn das Verhältnis von Polymerlösung zu Wasser mindestens 1:20, und insbesondere 1:50 bis 1:200 über einen Zeitraum von 16 bis 24 Stunden beträgt. Das VolumenVerhältnis der Polymer-Lösungen zu Wasser kann in einer ganz besonders bevorzugten Ausgestaltung der Erfindung 1:100 bei der Dialyse betragen (s. Tab. 5).

Polyfunktionelle Aminosäuren als Kryoprotektorenen, Porenbildner und als biologisch aktive Stoffe können in der Wundauflage mit einer optimalen Konzentration jeweils von 0,1 bis 15 % enthalten sein.

Als Wirkstoffe können vor allem SOD und Katalase eingesetzt werden, mit einer optimalen Menge von 0,1 bis 0,25 mg /cm² enthalten, die den Entzündungsprozess in der Wunde und die damit verbundenen Schmerzen reduzieren. Die Anwendung von Retinoiden führt zur Beschleunigung des Wundheilungsprozesses und zu einer Beschleunigung der Epithelisierung. Granulierungsphase.

Als Zusatzstoffe werden antibakterielle Stoffe z.B. Chlorhexidinbigluconat und / oder Polysept (bis 0,6 %), Hilfsstoffe aus der Gruppe der Polyalkohole z. B. Glyderin (10-30 %), hochmolekulare Stoffe zur Verbesserung der Adhäsion auf der Wunde und zur Beeinflussung der Ausscheidung von pharmazeutisch aktiven Stoffen z. B. Polyvinylalkohol und / oder Polyvinylpyrrolidon (4-10 %) verwendet (Tab. 4).

Zur Herstellung von Wundauflagen sollten zertifizierte Ausgangsstoffe verwendet werden, die keine toxischen oder infektiösen Bestandteile enthalten, z. B. Viren oder Prionen. Als zertifizierten Kollagen-Typ I, III kann man von der Firma Belkosin (Russland) und Gelatine nach Pharmakopoe (PB Gelatins, Gelita Europe, Rousselot a Sobel Company) und Chitosan (Sonat und Bioprogress (Russland), Hydagen® (Cognis AG), Chitosan (Protan, Inc.) einsetzen.

Insbesondere für Chitosan ist es vorteilhaft, das Ausgangsmaterial auszuwählen. Chitosan wird aus Chitin durch Deacetylierung und Depolymerisation gewonnen. Für medizinische Zwecke verwendet man Chitin mit einem Deacetylierungsgrad höher als 85 %. Chitosan ist heterogen bezüglich seines Molekulargewichtes, das je nach Herstellungsprozess zwischen 20 und 1000 kDa variieren kann. Wir haben gezeigt, dass Chitosan mit einer Molekularmasse niedriger als 100 kDa cytotoxische Wirkung zeigt bei der Züchtung von Fibroblasten auf Chitosan-Filmen. Es führt nicht zum Zelltod, sondern zur Unfähigkeit der Bildung eines Monolayers auf der Chitosanoberfläche. Die gezüchteten Fibroblasten haben eine ungewöhnliche Morphologie. Das niedrig molekulare Chitosan kann in diesem Fall in die Zellen eindringen und mit den Eiweißen vom Cytoskelett oder Cytoplasma reagieren.

Auf der anderen Seite weisen sowohl Chitosan mit einer Molekularmasse höher als 250 kDa als auch Glucosamin und Chitooligosaccharide mit einer Molekularmasse niedriger als 10 kDa keine cytotoxische Wirkung auf.

Eine ähnliche Abhängigkeit wurde auch bei der Darmflora gezeigt. Das niedrig molekulare Chitosan weist bakteriostatische und in mehreren Fällen bakterizide Eigenschaften auf.

Erfindungsgemäß sollte für eine bioverträgliche Wundauflage bevorzugt Chitosan mit einer Molekularmasse höher als 200 kDa verwendet werden.

Durch die erfindungsgemäß angewandte Dialyse (Elektrodialyse) wird dann niedrig molekulares Chitosan entfernt. Zusätzlich werden durch Dialyse ein Überschuss an polycarbonsäuren und andere niedrig molekulare Verunreinigungen der Ausgangsstoffe entfernt. Hinsichtlich Kollagen wurde gefunden, dass durch die erfindungsgemäße Dialyse eine fibrilläre Kollagenstruktur gebildet wird. Bei Gelatine führt die Dialyse überraschenderweise zur Erhöhung des Gewichtes von Spiralstrukturen (Erhöhung der optischen Aktivität).

Die Durchführung der Dialyse wird entsprechend der Zusammensetzung der Polymermischung vorgenommen. Der pH Wert sollte insbesondere zwischen 5,5 und 6,5 liegen. Um diesen zu erreichen, ist für eine Gelatine-Chitosan-Mischung eine einmalige kurzzeitige Dialyse notwendig. Für eine Kollagen-Chitosan-Lösung kann eine größere Menge Wasser benötigt werden.

Erfindungsgemäß wird bei der Dialyse eine Mischung der Polymerlösungen verwendet. Dadurch erhält man überraschenderweise eine Verbesserung der porösen Schwammstruktur. Die Dialyse von getrennten Polymerlösungen mit jeweils einem Polymer hingegen führt zu einer heterogenen Porenstruktur und ungleichmäßiger Oberfläche der Wundauflage.

Gemäß der RU 8608 B, 1998 ist bekannt, dass die Dialyse gegen Wasser von einer Kollagen-Chitosan-Mischung in Essigsäure zu einer Selbstgenerierung von Kollagen-Fibrillen führt, die durch die Anwesenheit von Chitosan in der Lösung, stark positiv beeinflusst wird. Überraschenderweise wird dieser Effekt durch den erfindungsgemäßen Einsatz der Polycarbonsäuren, die nicht Essigsäure, sind, erheblich verstärkt. In Fig. 1 und 2 ist die Bildung von Fibrillen einer Kollagen-Chitosan Mischung dargestellt, und zwar einmal gemäß RU 8608 (Essigsäure), Fig. 1 und einmal mit Polycarbonsäure (Erfindung) Fig. 2. Hierbei ist deutlich erkennbar, dass der Austausch von Essigsäure gegen Polycarbonsäure die Fibrillenbildung verstärkt, erkennbar anhand der deutlich höheren elektronischen Dichte der fibrillären Struktur.

In bevorzugten Ausführungsformen findet eine besonders effiziente Selbstorganisation von Kollagen-Fibrillen durch Dialyse bei einem Verhältnis von Polymerlösung zu Wasser von ca. 1 zu 100 und einer Dauer von 16 bis 24 Stunden statt.

Als Hilfsstoffe können niedrigmolekulare Verbindungen wie Glycerin und insbesondere hochmolekulare Polymere (Polyvinylalkohol, Polyvinylpyrrolidon und andere) eingesetzt werden. Dabei zeigte sich, dass überraschenderweise die Strukturen der Wundauflage noch weiter positiv beeinflusst werden und somit auch ihre Effizienz bei der Anwendung (z.B. Adhäsion zur Wundoberfläche). Außerdem sind sie in der Lage, die Wirkung von pharmazeutischen Präparaten zu verlängern. In Fig. 3 ist die prolongierte Wirkung von Superoxiddismutase bei Wundauflage ohne und mit Polyvinylalkohol (PVA) gezeigt.

Um die Stabilität der Wundauflagen gegenüber vorhandenen Enzymen in der Wunde zu erhöhen und die Einsatzzeit zu erhöhen, werden Vernetzer verwendet, die aus den Klassen der mono- oder bifunktionellen Agentien bestehen, oder die auf physikalischen Methoden basieren. Die Anwendung von vernetzenden Substanzen von Chitosan ist in der Literatur ausführlich beschrieben. Bevorzugt wird als effizienter Vernetzer Glutardialdehyd (GA) verwendet. Der Grund für diese Wahl beruht auf bekannten Erkenntnissen, dass bei der Herstellung von Wundauflagen die Restkonzentration von GA außerhalb ihrer cytotoxischen Wirkung liegt, nämlich weniger als 0,004 p.p.m (Beyer KI, et al. Chitosan-Collagen-Sponges. Estimation of the residual concentration of crosslinking agent. p.327-328. in. Proc.4th World Meeting ADRITELF, Florence 2002). Es zeigte sich, dass insbesondere bei Anwendung von GA in einer Konzentration von 0,01 % bis 0,03 % und pH-Wert von 5,2 bis 6,2 eine dreidimensionale vernetzte Chitosanmatrix gebildet werden kann. In dieser Matrix befinden sich freie Kollagenfibrillen oder Gelatine-Moleküle.

Erfindungsgemäß besteht die Herstellung von pharmazeutisch aktiven Wundauflagen insbesondere aus folgenden Schritten:
- Die benötigten Mengen von Chitosan und Kollagen (Gelatine) werden zusammen mit Polycarbonsäuren welche gleich oder verschieden sind in Wasser (jeweils getrennt) gelöst.
- Die entstehenden Polymermischungen werden gemeinsam dialysiert.
- Im Anschluss daran folgt die Zugabe von polyfunktionellen Aminosäuren, pharmakologischen Präparaten und Hilfsstoffen und/oder Zusatzstoffen.
- Der entstandenen Lösung werden Vernetzer hinzugegeben.
- Das entstandene Gel wird bei - 20 bis - 60°C eingefroren und letztendlich lyophilisiert.
- Das Schaummaterial wird konfektioniert und sterilisiert.

In den nachfolgenden Beispielen 1 bis 9 werden bevorzugte Ausführungsformen des erfindungsgemäßen Verfahrens dargestellt.

### Anwendung

Die erfindungsgemäßen Wundauflagen werden angewendet, indem das trockene Produkt in geeignete Formkörper geschnitten und auf die Wunden aufgelegt wird.

### Bioverträglichkeit und pharmakologische Wirkung der erfindungsgemäßen Wundauflagen (präklinische Studien)

Die präklinischen Studien für die erfindungsgemäßen Wundauflagen haben keine toxischen, reizenden oder sensibilisierenden Wirkungen gezeigt. Die Wundauflagen sind apyrogen, und laut ihren sanitär-hygienischen und toxikologischen Parametern entsprechen sie den geforderten Sicherheitsanforderungen an Medizinprodukten, die einen Kontakt zu menschlichen Wunden haben.

Experimentell wurde an Ratten mit künstlich erzeugten Verbrennungen mit Hilfe der erfindungsgemäßen Wundauflagen eine besser heilende Wirkung als mit Wundauflagen auf Alginatbasis gezeigt.

Ferner wurde auch eine antibakterielle Wirkung der erfindungsgemäßen Wundauflagen gezeigt.

### Klinische Studien

Die erfindungsgemäßen Wundauflagen wurden in randomisierten, kontrollierten, blinden, multizentrischen klinischen Studien an Patienten mit verschiedenen Wundäthiologien durchgeführt.

Die Wundauflagen wurden auf die Wunde aufgelegt und mit Mull oder anderen Fixierungsmitteln fixiert. Während der Aufnahme von Wundexsudat findet eine langsame Diffusion von in der Wundauflage enthaltenen biologisch und pharmakologisch aktiven Stoffen in die Wunde statt. Durch Kontakt mit der Wunde stimuliert Chitosan reparative Prozesse und beschleunigt die Wundheilung.

Die biologisch aktiven Stoffe und Kollagenfibrillen stimulieren die Zellaktivitäten z.B. die Immunreaktion. Langsam werden Teile der Wundauflage in der Wunde integriert und der Rest nach erfolgreicher Epithelisierung abgestoßen.

Bei infizierten und stark exsudierenden Wunden wurden die Wundauflagen täglich gewechselt. In gut granulierten Wundauflagen kann die Wundauflage auf der Wunde bis zur vollständigen Heilung verbleiben.

Die klinischen Studien haben eine hohe Effizienz bei der Heilung von infizierten und chronischen Wunden, Verbrennungen und chirurgischen Wunden an folgenden Wundheilungsphasen gezeigt:
- Entzündungs- und Reinigungsphase
- Granulationsphase
- Epithelisierungsphase

Aufgrund dieser Daten ist die erfindungsgemäße Wundauflage eine universelle Wundauflage, die bei allen Wundheilungsphasen eingesetzt werden kann. Die Wundheilungszeit ist im Vergleich zu üblichen Methoden um mindestens 20 % verkürzt.

Die detaillierte Beschreibung der praktischen Anwendung von den erfindungsgemäßen Wundauflagen sind in den Beispielen 10-11 aufgeführt. Insbesondere eignen sich erfindungsgemäße Auflagen bei der Behandlung von posttraumatischen und chirurgischen Wunden, ferner zur Beschleunigung der Heilung von Verbrennungen ersten bis dritten Grades, insbesondere auch bei der Heilung von infizierten oder chronischen Wunden verschiedener Ethiologie.

### Beispiele

Diese Erfindung ist nicht auf die dargestellten Beispiele begrenzt und kann weiter eingesetzt werden.

### Beispiel 1

In 150 ml wässriger Lösung von 1-%iger Glutaminsäure und 1-%iger Äpfelsäure wurden 3 g lyophilisiertes Kollagen hinzugegeben. Gleichzeitig wurde in 150 ml 1-%-iger Bernsteinsäure 3g Chitosan gelöst. Die beiden Lösungen wurden gemischt und im Laufe von 20 Stunden gegen Wasser dialysiert bis zu einem pH-Wert von 5,2. Zu dieser Lösung wurden 0,04g SOD, 0,03g Katalase und 0,06 ml 20 %-ige Chlorhexidinbigluconatlösung und 20 ml 3 %ige Argininlösung zugegeben. Danach wurde 5 ml 1,25 %ige Glutardialdehydlösung gegeben und anschließend wurde das entstandene Gel gefriergetrocknet.

Parallel zu dieser Zubereitung wurde die Wundauflage gemäß RU 8608 B, 1998 zubereitet
In 150 ml 3N Essigsäure wurden 3 g lyophilisiertes Kollagen gelöst. In 150 ml 2%-iger Essigsäure wurden 3g Chitosan gelöst.
Die beiden Lösungen wurden gemischt und im Laufe von 20 Stunden gegen Wasser bis zu einem pH-Wert von 5,2 dialysiert. Zu dieser Lösung wurden 0,04g SOD und 0,06 ml 20 %ige Chlorhexidinbigluconatlösung und 20 ml 3 %-ige Argininlösung zugegeben. Danach wurden 5 ml 1,25 %ige Glutardialdehydlösung hinzugefügt und anschließend wurde das entstandene Gel gefriergetrocknet.
Die Küvetten mit den gefrorenen Materialien wurden in einem Gefriertrockner bei - 35°C mit einer Gefrierungsgeschwindigkeit von 4°C ,10°C und 20 °C pro Stunde behandelt und anschließend getrocknet.
In allen Fällen standen poröse Materialien mit den in Tab. 1 und 2 dargestellten Charakteristiken.

**Tabelle 1: Vergleich der neuen Wundauflage nach erfindungsgemäßem Herstellungsverfahren mit einem Vergleichstyp.**

| Wundauflage | Einfriergeschwindigkeit °C/Stunde | Schwämme mit ungeeigneten Strukturen, in % |
|---|---|---|
| **(Vergleichstyp)** | 4 | 33 |
| | 10 | 72 |
| | 20 | 100 |
| **erfindungsgemäßes** | 4 | 0 |
| | 10 | 0 |
| | 20 | 2 |

**Tabelle 2: Vergleich von Wundauflagen (aus Beispiel 1) mit homogener (erfindungsgemäßes Verfahren) und nicht homogener (Vergleichstyp) poröser Struktur.**

| **Parameter** | **Maßeinheit** | **Wundauflagen mit homogener Porenstruktur** | **Wundauflagen mit nicht homogener Porenstruktur** |
|---|---|---|---|
| 1. Aussehen | - | Trockene poröse Struktur, geruchlos, hell-beige | Trockene poröse Struktur, geruchlos, hell-beige |
| 2. Elastizität | - | Der Schwamm lässt sich biegen zu einer Rolle | Der Schwamm zerbricht beim Zusammenrollen |
| 3. Abmessung | mm | 75 x 50 x 10 (± 2) | 60 x 40 x 8 (± 5) |
| 4. im Laufe von 24 Stunden freigesetzte SOD-Menge | %, an Trockengewicht | 7,4 ± 0.3 | 0,06 ± 0,04 |
| 5. spezif.Gewicht | g/cm³ | 0,014 ± 0,002 | 0,034 ± 0,015 |
| 6. pH-Wert des wässrigen Extraktes | pH | 5,8 ± 0,2 | 5,8 ± 0,2 |
| 8. Restfeuchtigkeit | % | 15,3 ± 0,5 | 38,2 ± 4,5 |
| 9. Adsorptionskapazität (Wasser) | % zum Trockengewicht | 7100 ± 500 | 1700 ± 1100 |
| 10. Gasdurchlässigkeit der Wundauflage | mg/cm²/Stunde | 4,5 ± 0,5 | < 0.1 |

### Beispiel 2

Im Beispiel 2 wird der Einfluss von Zusammensetzung und Konzentrationen von Polycarbonsäuren und Aminosäuren auf die Eigenschaften von Wundauflagen gezeigt. Die Herstellung der Wundauflagen wurde wie in Beispiel 1 dargestellt durchgeführt.

**Tabelle 3: Einfluss der Herstellungsbedingungen auf die Wundauflagen-Eigenschaften,**

| Nr | Carbonsäure (PKS) | Aminosäure (AS) | Chitosan/ PKS-Verhältnis in der Ausgangslösung | PKS/AS-Verhältnis in der Wundauflage | Ausschuss % | Eigenschaften | |
|---|---|---|---|---|---|---|---|
| | | | | | | Brüchigkeit (Härte) | Porosität |
| 1 | Bernsteinsäure | Glycin (8,5%) | 1:8 | 1:0,85 | 100 | hoch | groß |
| 2. | Bernsteinsäure | Glycin (7,3%) | 1:4 | 1:1,8 | <12 | hoch | groß |
| 3 | Bernsteinsäure | Glycin (7,3%) | 1:2 | 1:3.3 | <2 | niedrig | groß |
| 4. | Glutaminsäure | Glycin (7,3%) | 1:2 | 1:3,3 | 17 | niedrig | mittlere |
| 5 | Malonsäure | Glycin (7,3%) | 1:2 | 1:3,3 | 6 | niedrig | groß |
| 6 | GAS | Glycin (15%) | 1:2 | 1:7,5 | 100 | hoch | groß |
| 7 | GAS | Glycin (7,2%) | 1:2 | 1:3,3 | 0 | niedrig | groß |
| 8 | GAS | Glycin (3,2%) | 1:2 | 1:1,5 | <2% | niedrig | mittlere |
| 9 | GAS | Glycin (2,0%) | 1:2 | 1:0,9 | >70% | hoch | keine |
| 10 | Bernsteinsäure | Glycin (7,2%) | 1:1 | 1:6 | <2% | niedrig | groß |
| 11 | Bernsteinsäure | Glutamin (7,2%) | 1:1 | 1:6 | <5% | niedrig | groß |
| 12 | Bernsteinsäure | Alanin (6,6%) | 1:1 | 1:6 | <3% | niedrig | groß |
| 13 | Bernsteinsäure | Glycin (15%) | 1:0,5 | 1:15 | <10% | niedrig | mittlere |
| 14 | Bernsteinsäure. | Glycin (7,2%) | 1:1 | 1:3,6 | 0% | niedrig | mittlere |

| | | | | | | | |
|---|---|---|---|---|---|---|---|
| GAS: Bernsteinsäure und Äpfelsäure im Gewichtsverhältnis 1:1 | | | | | | | |

**Tabelle 4: Zusammensetzung (in Gew.%) von Wundauflagen mit verschiedenen Verhältnissen an Carbonsäuren und Aminosäuren.**

| Zusammensetzung Gew.% | Nr. entsprechend Tabelle 3 | | | | | | |
|---|---|---|---|---|---|---|---|
| | **1** | **2** | **3** | **4** | **5** | **6** | **7** |
| Chitosan | 57,0 | 36,4 | 44,3 | 44,3 | 36,8 | 35,8 | 18,0 |
| Kollagen | 19,0 | 36,4 | 44,3 | 44,3 | 36,8 | 35,8 | 56,0 |
| Bernsteinsäure* | 10,0 | 4,1 | 2,2 | | | | |
| Glutaminsäure* | | | | 2,2 | | | |
| Malonsäure* | | | | | 2,2 | | |
| Säuregemisch* | | | | | | 2,0 | 2,2 |
| Glutardialdehyd | 5,0 | 0,7 | 1.0 | 1.0 | 0,6 | 0,6 | 0,7 |
| SOD | 0,5 | 0,1 | 0,9 | 0,9 | 0,9 | 0,8 | 0,9 |
| Gebitan | | | | | | | |
| Glycerin | | 15,0 | | | 15,4 | 10 | 15,0 |
| Glycin | 8,5 | 7,3 | 7,3 | 7,3 | 7,3 | 15,0 | 7,2 |
| Glutamin | | | | | | | |
| Alanin | | | | | | | |
| Polyvinylalkohol | | | | | | | |

| | | | | | | | |
|---|---|---|---|---|---|---|---|
| * - Die Reste von Carbonsäuren in den Wundauflagen wurden nach entsprechenden Eichungen für Säuren in Lösungen berechnet | | | | | | | |

**Tabelle 4: (Fortsetzung) Zusammensetzung (in Gew.%) von Wundauflagen mit verschiedenen Verhältnissen an Carbonsäuren und Aminosäuren.**

| Zusammensetzung Gew.% | **Nr.** entsprechend Tabelle 3 | | | | | | |
|---|---|---|---|---|---|---|---|
| | **8** | **9** | **10** | **11** | **12** | **13** | **14** |
| Chitosan | 58,4 | 42,5 | 36,1 | 35,0 | 30,5 | 41,3 | 31,0 |
| Kollagen | 35,4 | 42,5 | 36,1 | 35,0 | 30,5 | 41,3 | 31,0 |
| Bernsteinsäure* | | | 1,2 | 1,2 | 1,1 | 1,0 | 2,1 |
| Glutaminsäure* | | | | | | | |
| Malonsäure* | | | | | | | |
| Säuregemisch* | 2,2 | 2,2 | | | | | 2,0 |
| Glutardialdehyd | 0,7 | 0,8 | 0,6 | 0,7 | 0,6 | 0,5 | 0,9 |
| SOD | 0,1 | 10 | 0,9 | 0,9 | 0,7 | 0,3 | 0,6 |
| Gebitan | | | | | | 0,6 | 0,2 |
| Glycerin | | | 17,9 | 20,0 | 30,0 | | 20 |
| Glycin | 3,2 | 2,0 | 7,2 | | | 15 | 7,2 |
| Glutamin | | | | 7,2 | | | |
| Alanin | | | | | 6,6 | | |
| Polyvinylalkohol | | | | | | | 5,0 |

### Beispiel 3

In 150 ml Lösung von 2%-iger Bernsteinsäure wurden 3 g lyophilisiertes Kollagen hinzugegeben. Gleichzeitig wurde in 150 ml 1-%iger Bernsteinsäure 3g Chitosan gelöst. Die beiden Lösungen wurden gemischt und im Laufe von 20 Stunden gegen Wasser bis zu einem pH-Wert von 5,2 dialysiert. Zu dieser Lösung wurden 0,04g SOD oder 0,03g Katalase und 20 ml 2%-ige Prolinlösung zugegeben.

Die entstandene Lösung wurde in zwei gleiche Teile aufgeteilt. In den einen Teil wurden 2,5 ml 1,25 %ige Glutardialdehydlösung gegeben. In den anderen Teil wurden 0,3 g Polyvinylalkohol in 20 ml Wasser gegeben. Danach wurden 2,5 ml 1,25 %ige Glutardialdehydlösung gegeben. Die entstandenen Materialien wurden bei -35 °C eingefroren und gefriergetrocknet, In den fertigen Wundauflagen wurde SOD in einer Konzentration von 0,6 % vom Trockengewicht oder 0,1 mg pro cm² nachgewiesen. Die spezifische Dichte betrug 0,014 +/- 0,001 g pro cm³. Die Adsorptionskapazität für Wasser betrug 7100 +/- 500 %. Die Polyvinylalkohol enthaltenden Wundauflagen hatten gleichmäßige und etwas kleinere Poren. Die Ausscheidung von SOD wurde nach Bestimmung der Aktivität von SOD durch Verzögerung der Quercitinoxidation (Glazer A.N. et al. Methods Enzymol. 1990, 186,161-168) bestimmt. Die Wundauflagen, die Polyvinylalkohol enthalten, haben eine verzögerte Ausscheidung von SOD bei Rehydratation in Wasser gezeigt (Abb. 3).

### Beispiel 4

In 300 ml 2%-ige Apfelsäure wurden je 3 g Chitosan und Kollagen gelöst. Die weitere Behandlung findet wie in Beispiel 1 beschrieben statt. Danach wurden in die Polymerlösung 15 ml Carotin enthaltende Liposomen gegeben. Das liposomale ß-Carotin wurde wie im folgenden Abschnitt beschrieben hergestellt:

In 10 ml Chloroform wurden 1,2 g Phospholipide (Nattermann Phospholipid GmbH) und 0,3 g ß-Carotin zugegeben. Zu der entstandenen Lösung wurde 8,4 g Polyvinylalkohol gegeben. Die entstandene Suspension wurde intensiv gerührt und

in eine Küvette gegossen. Das Chloroform wurde in einem Vakuumschrank entfernt. Das trockene Produkt wurde gemahlen und danach zu 150 ml Phosphatpuffer mit pH 6,8 bis 7,4 gegeben und intensiv über einen Zeitraum von 30 Minuten gerührt. Dadurch entstanden multischichtige Liposomen. Um die Liposomengröße zu verkleinern ud gleichzeitig zu sterilisieren, wurden sie mit Ultraschall (150 -200 W) bearbeitet.

Die 0,2% ß-Carotin und 2 % Phospholipide enthaltenden Liposomen wurden zur Kollagen-Chitosan-Lösung hinzugefügt. Dazu wurden 0,02 ml 20 %ige Chlorhexidinbigluconat und 0,006 ml Polysept und bis 30 % plastifizierender Stoff z.B. Glycerin gegeben. Die Vernetzung und Gefriertrocknung erfolgten wie in Beispiel 1 dargestellt. Das fertige Produkt wurde geschnitten, verpackt und markiert. Anschließend erfolgte eine Gammasterilisation mit einer Dosis von 1,5 Mrad.

Die Carotin enthaltenden Wundauflagen werden meistens für die Heilung von chronischen Wunden eingesetzt. In allen durchgeführten Studien wurde eine stimulierende Wirkung auf die Epithelisierungsprozesse nachgewiesen.

### Beispiel 5

3 g Gelatine wurden in 150 ml 0,1 %-iger Milchsäure quellen gelassen und anschließend bei 70°C gelöst. In 130 ml Wasser wurden 3 g Chitosan mit einer Molekularmasse von 350 kDa und Deacetylierungsgrad von mehr als 85 % und 0,7 g Bernsteinsäure bis zur vollständigen Lösung von Chitosan gelöst. Die beiden Lösungen wurden gemischt und anschließend gegen Wasser im Verhältnis 1 zu 100 dialysiert. Zu dieser Lösung wurden 20 ml 20 % ige Taurinlösung gegeben. Die Vernetzung und anschließende Gefriertrocknung wurden ähnlich wie in Beispiel 1 durchgeführt. Die gewonnenen Schwämme haben eine spezifische Dichte von 0,012 bis 0,016 g/cm³. Die Sorptionskapazität für Wasser beträgt von 4000 bis 6000 %.

### Beispiel 6

Zu 900 ml apyrogenem deionisiertem Wasser wurden 10 g Chitosan und 5 g Milchsäure hinzugegeben. Die Lösung wurde bis zur vollständigen Auflösung von Chitosan gerührt.

Zu 900 ml apyrogenem deionisiertem Wasser wurden 0,9 g Pyrolidonkarconsäure und 10 g Gelatine gegeben und durch Erhitzen auf 70°C gelöst.

Die beiden Lösungen wurden zusammengemischt und dialysiert Anschließend wurden 40 ml 20% ige Glycin-Lösung zugegeben. Die Vernetzung und Gefriertrocknung wurde wie in Beispiel 1 dargestellt durchgeführt.

Die gewonnenen Schwämme haben eine spezifische Dichte von 0,020 bis 0,022 g/cm³. Die Sorptionskapazität für Wasser beträgt von 4000 bis 5000 %.

Das fertige Produkt wurde geschnitten, verpackt und markiert. Anschließend erfolgte eine Gammasterilisation mit einer Dosis von 1,5 Mrad.

### Beispiel 7

10 g Chitosan wurden in 500 ml 10 g Bernsteinsäure enthaltendem deionisiertem Wasser gelöst.

10 g Kollagen wurden in 500 ml 10 g Bernsteinsäure enthaltendem deionisiertem Wasser gelöst.

Die beiden Lösungen wurden vermischt und anschließend gegen Wasser in einem Verhältnis 1 zu 100 dialysiert.

2 g Gelatine wurden in 100 ml Wasser quellen gelassen und anschließend bei 70 °C gelöst. Die erhaltene Lösung wurde bei 0,5 bar 45 Minuten 125°C autoklaviert. Die autoklavierte Gelatine wurde dann mit Chitosan-Kollagen-Lösung gemischt. Die durch Thermohydrolyse entstandenen Aminosäuren und Peptide sind in der Lage, die Polymerstrukturen der Wundauflage zu stabilisieren.

Zu dieser Lösung wurden 0,1 ml Chlorhexidinbigluconat gegeben. Die Vernetzung und Gefriertrocknung erfolgten wie in Beispiel 1 dargestellt.

Die gewonnenen Schwämme haben eine spezifische Dichte von 0,012 bis 0,016 g/cm³. Die Sorptionskapazität für Wasser beträgt von 5000 bis 8000 %.

Das fertige Produkt wurde geschnitten, verpackt und markiert. Anschließend erfolgte eine Gammasterilisation mit einer Dosis von 1,5 Mrad.

### Beispiel 8

10 g Chitosan wurden in 500 ml 10 g Bernsteinsäure enthaltendem deionisiertem Wasser gelöst.

10 g Kollagen wurden in 500 ml 10 g Bernsteinsäure enthaltendem deionisiertem Wasser gelöst.

Die beiden Lösungen wurden vermischt und anschließend gegen Wasser in verschiedenen Verhältnissen Wasser zur Polymerlösung dialysiert.

Zu dieser Lösung wurden 0,05 g SOD und 80 ml 2 % ige Glycinlösung gegeben. Die Vernetzung und Gefriertrocknung erfolgten wie in Beispiel 1 dargestellt.

In Tabelle 5 sind die Charakteristiken der hergestellten Produkte dargestellt.

**Tabelle 5: Einfluss der Dialyse auf die Eigenschaften von Wundauflagen.**

| Nr. | Lösung-Wasser-Verhältnis | Dialyse Dauer, Stunde | pH-Wert der Ausgangslösung | pH-Wert nach Dialyse | pH-Wert des Wasserauszuges | SOD-Aktivität, in % zu native SOD |
|---|---|---|---|---|---|---|
| 1 | 1:20 | 20 | 4,2 | 4,5 | 4,6 | 42 ± 10 |
| 2 | 1:50 | 20 | 4,2 | 4,7 | 4,9 | 68 ± 10 |
| 3 | 1:100 | 20 | 4,2 | 5,2 | 5,6 | 87 ± 10 |
| 4 | 1:100 | 10 | 4,2 | 4,7 | 4,8 | 63 ± 10 |
| 5 | 1:200 | 20 | 4,2 | 5,4 | 6,0 | 90 ±10 |

Die Aktivität von SOD wurde durch die Inhibierung der Quercitin-Reaktion bestimmt. Die höchste Aktivität von SOD zeigten Wundauflagen, die durch Dialyse in einem Verhältnis 1 : 100 und 1: 200 und 20 Stunden Dauer vorbereitet wurden.

### Beispiel 9

Die Polymerlösungen wurden wie in Beispiel 8 dargestellt hergestellt.

Die Wundauflagen wurden mit zwei verschiedenen Verfahren hergestellt.

In dem einen Verfahren wurden Chitosan- und Kollagen-Lösungen vermischt und anschließend dialysiert.

Im anderen Verfahren wurden die Chitosan- und Kollagen-Lösungen zunächst separat dialysiert und dann vermischt.

Anschließend wurden zu den erhaltenen Lösungen 0,1 ml Chlorhexidinbigluconat, 40 ml 2% iges Arginin und 2 g Glycerin gegeben.

Die Vernetzung und Gefriertrocknung erfolgten wie in Beispiel 1 dargestellt.

Die entstandenen Wundauflagen waren von Aussehen unterschiedlich. Die Wundauflagen nach dem ersten Verfahren waren elastisch und mit guter Porosität, während die Wundauflagen nach dem zweiten Verfahren nicht elastisch, polydispers und mit welliger Oberflächenstruktur waren.

### Die klinische Prüfung der erfindungsgemäßen Wundauflage.

### Beispiel 10

Für die klinische Prüfung wurden sterile Wundauflagen, hergestellt wie in Beispiel 1, verwendet.

Die klinischen Studien wurden an 29 Patienten im Alter von 26 bis 92 Jahren für die Behandlung von Wunden verschiedener Ethiologie (chronische Wunden, Decubitus, posttraumatische Wunden), die sich in der Granulationsphase befanden, eingesetzt.

Vor der Anwendung der Wundauflagen wurden die Wunden standardmäßig gereinigt.

Die Verpackung der Wundauflagen wurde mit sterilen Scheren geöffnet. Die Wundauflagen wurden dann entsprechend der Wundgröße mit einem Überstand von 0,5 cm geschnitten. Die ausgeschnittene Wundauflage wurde fest auf den Wundboden gedrückt und zusätzlich mit einem üblichen Fixierungsmittel fixiert. Durch Aufnahme von Wundexsudat waren die Wundauflagen weich und zeigten eine gute Adhäsion zur Wunde. Bei Behandlung von chronischen Wunden mit Superoxiddismutase enthaltenden Wundauflagen, wurde ein normaler Verlauf des Wundheilungsprozesses beobachtet:

Der Wundboden wurde von Fibrinresten gereinigt und durch rosanes feinkörniges Granulationsgewebe ausgefüllt.

In der Epithelisationsphase wurde die Wundauflage auf der Wunde bis zur vollständigen Heilung behalten. Die epithelisierte Wundoberfläche war nach der Abnahme des Verbandes ohne Hyperkertose (ohne größere Narben).

Bei der Behandlung von posttraumatischen Wunden (nach Autoplastik) wurden die Wundauflagen unmittelbar nach Entstehung der Wunde aufgebracht. Dabei wurde eine blutstillende Wirkung beobachtet. Die Wundauflagen wurden auf der Wunde bis zur vollständigen Epithelisierung behalten.

Im Vergleich zur Kontrolle wurde ein beschleunigter Wundheilungsprozess beobachtet. Vier bis fünf Tage früher trat eine vollständige Epithelisierung bei Verbrennungen und posttraumatischen Wunden ein. Bei der Heilung von chronischen Wunden wurde eine 50 % ige Verkleinerung der Oberfläche und Tiefe der Wunde durchschnittlich 10 - 15 Tage früher im Vergleich zur Kontrolle nachgewiesen (Tab. 6).

Bei der Anwendung der erfindungsgemäßen Wundauflagen wurde die Häufigkeit des Wechselns von Wundauflagen um ca. 30 % verringert. Dies wurde auch von den Patienten positiv aufgenommen.

Auf der Basis klinischer Beobachtungen wurde eine stimulierende Wirkung auf reparative Prozesse in der Wunde gezeigt, insbesondere bei älteren Patienten mit einem verzögerten Heilungsprozess. Es wurde keine Infektion oder Verschlechterung des Heilungsprozesses beobachtet.

Bei der Anwendung der erfindungsgemäßen Wundauflagen wurden von den Patienten keine Beschwerden bezüglich Schmerzen, Juckreiz oder Brennen geäußert.

Ferner wurde eine effektive antihypoxische Wirkung bei den erfindungsgemäßen Wundauflagen nachgewiesen. Diese antihypoxische Wirkung beruht wahrscheinlich auf der Anwesenheit von Bernsteinsäure und Arginin, die in der Lage sind, den Atmungsprozess unabhängig von der Sauerstoffzufuhr in dem Gewebe zu normalisieren.

Bei der Kontrolle wurde eine große Anzahl von unangenehmen Nebenwirkungen (Schmerzen, Brennen und Juckreiz) beobachtet - insbesondere in den ersten Minuten nach der Behandlung. Ferner wurde auch der unangenehme Essigsäuregeruch als störend wahrgenommen.

**Tabelle 6:**

| Die Effizienz der Wundheilung nach Anwendung von erfindungsgemäßen Wundauflagen und Prototypen an Patienten mit chronischen- und posttraumatischen Wunden. | | | | | | | | |
|---|---|---|---|---|---|---|---|---|
| Diagnose | Anzahl der Patienten | Wundauflage | **Ergebnisse** | | | | | |
| | | | Adhäsion zur Oberfläche | Häufigkeit des Wundauflagenwechsels | Erste Granulation | Verträglichkeit der Wundauflage | Ende der Epithelisierung | Nebenwirkungen |
| **A** | 19 | erfindungsgemäß | +++ | alle 2-3 Tage | am 3.-5. Tag | Schmerz 0 Brennen 1 Juckreiz 0 | am 8. - 9. Tag | keine |
| | | Vergleich | ++ | alle 2-3 Tage | am 4.-7. Tag | Schmerz 2 Brennen 2 Juckreiz 0 | am 12. - 15. Tag | Allergische Dermatitis - 1 |
| **B** | 12 | erfindungsgemäß | ++ | alle 2 Tage | am 5.- 6. Tag | Schmerz 0 Brennen 0 Juckreiz 0 | 50%-ige Verkleine rung der Wunde am 30. - 35. Tag | keine |
| | | Vergleich | ++ | alle 2 Tage | am 8.- 10. Tag | Schmerz 3 Brennen 2 Juckreiz 3 | am 40. Tag | Allergische Dermatitis - 3 |

### A- Posttraumatische Wunden (u.a. nach autologer Hautplastik), B- Chronische Wunden durch CVI

### Beispiel 11

### Die Heilung von infizierten Wunden mit Wundauflagen aus Beispiel 3

Die klinischen Studien wurden an zwölf Patienten im Alter von 17 bis 28 Jahren mit schlecht heilenden Wunden, die auf Grund von eitrigen und entzündlichen Prozessen entstanden (Phlegmone, Venenentzündung u.a.) sind, durchgeführt. Als Kontrolle wurden Wundauflagen aus Beispiel 1 auf Essigsäurebasis eingesetzt. Nach der Entfernung von Eiter und nekrotischem Gewebe wurden die Wundauflagen wie in Beispiel 11 mit täglichem Wechsel der Wundauflagen angewendet.

Die klinischen Beobachtungen wurden in den ersten 10 bis 15 Tagen durchgeführt. Schon nach 5 bis 7 Tagen bei Anwedung der erfindungsgemäßen Wundauflagen wurde der Wundboden von Fibrinresten gereinigt und durch rosanes feinkörniges Granulationsgewebe ausgefüllt.

In den folgenden 4 bis 5 Tagen verkleinerte sich die Wundoberfläche erheblich, und die Gewebedefekte wurden mit frischem Granulationsgewebe aufgefüllt.

Bei acht Patienten wurde eine frühere Insel- und Randepithelisierung ca. drei Tage früher als bei der Kontrolle beobachtet.

Die Ergebnisanalyse u. a. zytologische Beobachtungen hat gezeigt, dass die Anwendung der erfindungsgemäßen Wundauflagen zu einer Stimulierung der leukozytären Reaktion (ca. 35 % höher als Kontrolle) und zur Beschleunigung der Granulations- und Epithelisierungsprozesse geführt hat. Es wurden keine negativen Nebenwirkungen und Reaktionen beobachtet.

## Patentansprüche

1. Wundauflage, **dadurch gekennzeichnet, dass** sie von 19 bis 56% eines oder mehrerer Struktureller Eiweiße, ausgewählt aus Kollagen, Gelatine, Derivaten oder Mischungen hiervon, 18 bis 58% eines oder mehrerer struktureller Polysaccharide, ausgewählt aus Chitosan, Chitosan-Derivaten oder Mischungen hiervon, 0,5 bis 10 % Polycarbonsäuren/Carbonsäuren, enthaltend zusätzlich zu einer Carboxylgruppe eine oder mehrere funktionelle Gruppen, ausgewählt aus Carboxy-, Hydroxy- Amino-, ausgewählt aus Milchsäure, Äpfelsäure, Bernsteinsäure, Malonsäure, Fumarsäure, Ascorbinsäure, Glutaminsäure, Salicylsäure, Pyrrolidoncarbonsäure oder Mischungen hiervon, 0,1 bis 15 % polyfunktionelle Aminosäuren/Aminosäuren, ausgewählt aus Arginin, Methionin, Prolin, Taurin, Glycin, Alanin, Cystein, N-Acetylcystein oder Mischungen hiervon, 0 bis 10% wirksame Substanzen, 0 bis 30% Hilfs- und / oder Zusatzstoffe, und 0,2 bis 5% Vernetzer aufweist.

2. Wundauflage gemäß Anspruch 1, **dadurch gekennzeichnet, dass** die Polycarbonsäuren/Carbonsäuren ausgewählt sind aus Bernsteinsäure, Glutaminsäure, Malonsäure, Äpfelsäure, Pyrrolidoncarbonsäure, Milchsäure.

3. Wundauflage gemäß Anspruch 1 oder 2, **dadurch gekennzeichnet, dass** die polyfunktionellen Aminosäuren/Aminosäuren ausgewählt sind aus Glycin, Glutamin, Alanin, Arginin, Prolin, Taurin.

4. Verfahren zur Herstellung einer Wundauflage, enthaltend 19 bis 56% eines oder mehrerer Struktureller Eiweiße, ausgewählt aus Kollagen, Gelatine, Derivaten oder Mischungen hiervon, 18 bis 58% eines oder mehrerer struktureller Polysaccharide, ausgewählt aus Chitosan, Chitosan- Derivaten oder Mischungen, 0,5 bis 10 % Polycarboncarbonsäuren/Carbonsäuren enthaltend zusätzlich zu einer Carboxylgruppe eine oder mehrere funktionelle Gruppen, ausgewählt aus Carboxy-, Hydroxy- Amino-, ausgewählt aus Milchsäure, Äpfelsäure, Bernsteinsäure, Malonsäure, Fumarsäure, Ascorbinsäure, Glutaminsäure, Salicylsäure, Pyrrolidoncarbonsäure oder Mischungen hiervon, 0,1 bis 15 % polyfunktionelle Aminosäuren/Aminosäuren, ausgewählt aus Arginin, Methionin, Prolin, Taurin, Glycin, Alanin, Cystein, N-Acetylcystein oder Mischungen hiervon, 0 bis 10% Wirksubstanzen, 0,2 bis 5 % Vernetzer, 0 bis 30 % Hilfs- und/oder Zusatzstoffe, **dadurch gekennzeichnet, dass** man einer wässrigen Lösung des Polysaccharides die Polycarbonsäure(n)/Carbonsäure(n) hinzufügt und einer wässrigen Lösung eines strukturellen Eiweißes die gleiche oder eine andere Polycarbonsäure(n)/Carbonsäure(n) hinzufügt, anschließend beide Polymerlösungen gemeinsam dialysiert und sodann polyfunktionelle Aminosäure(n)/Aminosäure(n), und ggf. Wirkstoffe, Vernetzer, Zusatz- und /oder Hilfsstoffe der dialysierten Reaktionsmischung hinzufügt.

5. Verfahren gemäß Anspruch 4, **dadurch gekennzeichnet, dass** als strukturelle Eiweiße Kollagen verschiedenen Ursprungs verwendet wird.

6. Verfahren gemäß Anspruch 5 **dadurch gekennzeichnet, dass** als strukturelle Eiweiße Gelatine Typ A und Typ B verwendet werden.

7. Verfahren gemäß Anspruch 6, **dadurch gekennzeichnet, dass** hochmolekulare Gelatine mit einem Bloom-Wert höher als 200 verwendet wird.

8. Verfahren gemäß einem der Ansprüche 4 bis 7, **dadurch gekennzeichnet, dass** als Polysaccharid Chitosan, dessen wasserlöslichen Derivate oder Mischungen verwendet werden.

9. Verfahren gemäß einem der Ansprüche 4 bis 8, **dadurch gekennzeichnet, dass** Chitosan mit einer Molekularmasse höher als 200 kDa verwendet wird.

10. Verfahren gemäß einem der Ansprüche 4 bis 9, **dadurch gekennzeichnet, dass** als Polycarbonsäure/Carbonsäure Äpfelsäure, Bernsteinsäure, Malonsäure, Glutaminsäure, Pyrrolidoncarbonsäure oder Mischungen hiervon verwendet werden.

11. Verfahren gemäß Anspruch 4, **dadurch gekennzeichnet, dass** Polycarbonsäuren/Carbonsäuren zu hochmolekularen Stoffen in einem Verhältnis 1 : 4 bis 2 : 1 verwendet werden.

12. Verfahren gemäß Anspruch 4 bis 11, **dadurch gekennzeichnet, dass** die Lösungen von strukturellen Polysacchariden, insbesondere Chitosan und strukturellen Eiweißen mindestens 12 Stunden vor der Dialyse zusammengemischt werden.

13. Verfahren gemäß Anspruch 4 bis 12 **dadurch gekennzeichnet, dass** die Dialyse gegen Wasser in einem Volumenverhältnis Polymerlösung zu Wasser von mindestens 1:100 im Laufe von mehr als 16 Stunden abläuft.

14. Verfahren gemäß Anspruch 4 bis 13 **dadurch gekennzeichnet, dass** in die dialysierten Lösungen die polyfunktionellen Aminosäuren/Aminosäuren_gegeben werden.

15. Verfahren gemäß Anspruch 14 **dadurch gekennzeichnet, dass** als Aminosäuren Arginin, Prolin, Taurin, Glycin, Alanin, Glutamin oder Mischungen hiervon verwendet werden.

16. Verfahren gemäß Anspruch 4, **dadurch gekennzeichnet, dass** als bifunktioneller Vernetzer Glutardialdehyd verwendet wird.

17. Verfahren gemäß Anspruch 4 bis 16 **dadurch gekennzeichnet, dass** als pharmakologisch aktiver Stoff Superoxiddismutase und/oder Katalase verschiedenen Ursprungs verwendet wird.

18. Verfahren gemäß Anspruch 17, **dadurch gekennzeichnet, dass** Superoxiddismutase und/oder Katalase in einer Konzentration von 0,001 bis 0,1 % zur Polymerbasis verwendet wird.

19. Verfahren gemäß Anspruch 4 bis 17 **dadurch gekennzeichnet, dass** als pharmakologisch aktiver Stoff ß-Carotin verschiedenen Ursprungs verwendet wird.

20. Verfahren gemäß Anspruch 19, **dadurch gekennzeichnet, dass** als pharmakologisch aktiver Stoff ß-Carotin in liposomaler Form verwendet wird.

21. Verfahren gemäß Anspruch 19 oder 20 **dadurch gekennzeichnet, dass** ß-Carotin in einer Konzentration von 0,001 bis 0,05 % zur Polymerbasis verwendet wird.

22. Verfahren gemäß der Anspruche 4 bis 21, **dadurch gekennzeichnet, dass** als Hilfsstoffe antibakterielle Stoffe, ausgewählt aus Chlorhexidin, Polysept, Polyhexanid, Plastifikatoren, hochmolekulare Stoffe verwendet werden, die die Adhäsion zur Wundoberfläche gewährleisten und/oder Hilfsstoffe, die die Ausscheidung von pharmazeutisch aktiven Stoffen beeinflussen, verwendet werden.

23. Verfahren gemäß Anspruch 22 **dadurch gekennzeichnet, dass** antibakterielle Stoffe in einer Konzentration von 0,01 bis 0,6 % zur Polymerbasis verwendet werden.

24. Verfahren gemäß Anspruch 22 oder 23, **dadurch gekennzeichnet, dass** die Zusatz/Hilfsstoffe zu dem Dialysat in einer Konzentration von 10 - 30 % gegeben werden.

25. Verfahren gemäß Anspruch 24, **dadurch gekennzeichnet, dass** als Hilfsstoffe Polyvinylalkohol und Polyvinylpyrrolidon verwendet werden.

26. Verwendung einer Wundauflage gemäß Anspruch 1 bis 3 oder herstellbar gemäß Anspruch 4 bis 25 zur Herstellung eines Mittels für die beschleunigte Heilung von posttraumatischen und chirurgischen Wunden.

27. Verwendung einer Wundauflage gemäß Anspruch 1 bis 3 oder herstellbar gemäß Anspruch 4 bis 25 zur Herstellung eines Mittels für die beschleunigte Heilung von Verbrennungen ersten bis dritten Grades.

28. Verwendung einer Wundauflage gemäß Anspruch 1 bis 3 oder herstellbar gemäß Anspruch 4 bis 25 zur Herstellung eines Mittels für die beschleunigte Heilung von infizierten oder chronischen Wunden verschiedener Ethiologie.

29. Verwendung einer Wundauflage gemäß Anspruch 1 bis 3 oder herstellbar gemäß Anspruch 4 bis 25 zur Herstellung eines Mittels für die beschleunigte Heilung von posttraumatischen und chirurgischen, infizierten, chronischen Wunden oder von Verbrennungen.

## Claims

1. A wound dressing, **characterized in that** it has from 19 to 56% of one or more structural proteins, selected from collagen, gelatin, derivatives or mixtures thereof, 18 to 58% of one or more structural polysaccharides, selected from chitosan, chitosan derivatives or mixtures thereof, 0.5 to 10% of polycarboxylic acids/carboxylic acids containing in addition to a carboxyl group, one or more functional groups selected from carboxy, hydroxy, amino groups, selected from lactic acid, malic acid, succinic acid, malonic acid, fumaric acid, ascorbic acid, glutamic acid, salicylic acid, pyrrolidone-carboxylic acid or mixtures thereof, 0.1 to 15% of polyfunctional amino acids/amino acids, selected from arginine, methionine, proline, taurine, glycine, alanine, cysteine, N-acetylcysteine or mixtures thereof, 0 to 10% of active substances, 0 to 30% of adjuvants and/or additives, and 0.2 to 5% of cross-linking agents.

2. The wound dressing according to claim 1, **characterized in that** the polycarboxylic acids/carboxylic acids are selected from succinic acid, glutamic acid, malonic acid, malic acid, pyrrolidone-carboxylic acid, lactic acid.

3. The wound dressing according to claim 1 or 2, **characterized in that** the polyfunctional amino acids/amino acids are selected from glycine, glutamine, alanine, arginine, proline, taurine.

4. A method for preparing a wound dressing, containing 19 to 56% of one or more structural proteins, selected from collagen, gelatin, derivatives or mixtures thereof, 18 to 58% of one or more structural selected from chitosan, chitosan structural polysaccharides, selected from chitosan, chitosan derivatives or mixtures thereof, 0.5 to 10% of polycarboxylic acids/carboxylic acids containing in addition to a carboxyl group, one or more functional groups selected from carboxy, hydroxy, amino groups, selected from lactic acid, malic acid, succinic acid, malonic acid, fumaric acid, ascorbic acid, glutamic acid, salicylic acid, pyrrolidone-carboxylic acid or mixtures thereof, 0.1 to 15% of polyfunctional amino acids/amino acids, selected from arginine, methionine, proline, taurine, glycine, alanine, cysteine, N-acetylcysteine or mixtures thereof, 0 to 10% of active substances, 0.2 to 5% of cross-linking agents, 0 to 30% of adjuvants and/or additives, **characterized in that** the polycarboxylic acid(s)/carboxylic acid(s) is(are) added to an aqueous solution of the polysaccharide, and the same or another of the polycarboxylic acid(s)/carboxylic acid(s) is(are) added to an aqueous solution of a structural protein, both polymer solutions are subsequently dialyzed together and ten polyfunctional amino acid(s)/amino acid(s), and optionally active substances, cross-linking agents, additives and/or adjuvants are added to the dialyzed reaction mixture.

5. The method according to claim 4, **characterized in that** collagen of various origin is used as structural proteins.

6. The method according to claim 5, **characterized in that** gelatins of type A or of type B are used as structural proteins.

7. The method according to claim 6, **characterized in that** high molecular gelatin with a bloom value above 200 is used.

8. The method according to one of claims 4 to 7, **characterized in that** chitosan, its water-soluble derivatives or mixtures are used as polysaccharides.

9. The method according to one of claims 4 to 8, **characterized in that** chitosan with a molecular mass above 200 kDa is used.

10. The method according to one of claims 4 to 9, **characterized in that** malic acid, succinic acid, malonic acid, glutamic acid, pyrrolidone-carboxylic acid or mixtures thereof are used as a polycarboxylic acid/carboxylic acid.

11. The method according to claim 4, **characterized in that** polycarboxylic acids/carboxylic acids are used in a ratio from 1:4 to 2:1, relatively to the high molecular substances.

12. The method according to claim 4 to 11, **characterized in that** the solutions of structural polysaccharides, in particular chitosan and structural proteins, are mixed together at least 12 hours before the dialysis.

13. The method according to claim 4 to 12, **characterized in that** the dialysis against water takes place in a volume ratio of polymer sol ution to water of at least 1:100 over more than 16 hours.

14. The method according to claim 4 to 13, **characterized in that** the polyfunctional amino acids/amino acids are added into the dialyzed solutions.

15. The method according to claim 14, **characterized in that** arginine, proline, taurine, glycine, alanine, glutamine or mixtures thereof are used as amino acids.

16. The method according to claim 4, **characterized in that** glutaric dialdehyde is used as a bifunctional cross-linking agent.

17. The method according to claim 4 to 16, **characterized in that** super oxide dismutase and/or catalase of various origin is used as a pharmacologically active substance.

18. The method according to claim 17, **characterized in that** the super oxide dismutase and/or catalase is used in a concentration from 0.001 to 0.1%, based on the polymer.

19. The method according to claim 4 to 17, **characterized in that** β-carotene of various origin is used as a pharmacologically active substance.

20. The method according to claim 19, **characterized in that** β-carotene in liposomal form is used as a pharmacologically active substance.

21. The method according to claim 19 or 20, **characterized in that** β-carotene is used in a concentration from 0.001 to 0.05%, based on the polymer.

22. The method according to claim 4 to 21, **characterized in that** antibacterial substances selected from chlorhexidine, polysept, polyhexanide, plasticizers, high molecular substances are used as adjuvants, which guarantee adhesion to the wound surface and/or adjuvants which influence the elimination of pharmaceutically active substances, are used.

23. The method according to claim 22, **characterized in that** antibacterial substances are used in a concentration from 0.01 to 0.6%, based on the polymer.

24. The method according to claim 22 or 23, **characterized in that** the additives/adjuvants are added to the dialyzate in a concentration from 10 to 30%.

25. The method according to claim 24, **characterized in that** polyvinyl alcohol and polyvinylpyrrolidone are used as adjuvants.

26. The use of a wound dressing according to claim 1 to 3, or which may be prepared according to claim 4 to 25 for preparing an agent for accelerated healing of post-traumatic and surgical wounds.

27. The use of a wound dressing according to claim 1 to 3 or which may be prepared according to claim 4 to 25 for preparing an agent for accelerating healing of bums of the first to the third degree.

28. The use of a wound dressing according to claim 1 to 3 or which may be prepared according to claim 4 to 25 for preparing an agent for accelerating healing of infected or chronic wounds of various etiology.

29. The use of a wound dressing, according to claim 1 to 3 or which may be prepared according to claim 4 to 25 for preparing an agent for accelerating healing of post-traumatic and surgical, infected, chronic wounds or of burns.

## Revendications

1. Pansement, **caractérisé en ce qu'**il présente 19 à 56% d'une ou de plusieurs protéines structurelles, choisies parmi le collagène, la gélatine, les dérivés ou les mélanges de ceux-ci, 18 à 58% d'un ou de plusieurs polysaccharides structurels, choisis parmi le chitosane, les dérivés de chitosane ou leurs mélanges, 0,5 à 10% d'acides polycarboxyliques/ carboxyliques, contenant en plus d'un groupe carboxyle un ou plusieurs groupes fonctionnels, choisis parmi les groupes carboxy, hydroxy, amino, choisis parmi l'acide lactique, l'acide malique, l'acide succinique, l'acide malonique, l'acide fumarique, l'acide ascorbique, l'acide: glutamique, l'acide salicylique, l'acide pyrrolidonecarboxylique ou leurs mélanges, 0,1 à 15% d'aminoacides polyfonctionnels/aminoacides, choisis parmi l'arginine, la méthionine, la proline, la taurine, la glycine, l'alanine, la cystéine, la N-acétylcystéine ou leurs mélanges, 0 à 10% de substances actives, 0 à 30% d'adjuvants et/ou d'additifs, et 0,2 à 5% de réticulant.

2. Pansement selon la revendication 1, **caractérisé en ce que** les acides polycarboxyliques/carboxyliques sont choisis parmi l'acide succinique, l'acide glutamique, l'acide malonique, l'acide malique, l'acide pyrrolidonecarboxylique, l'acide lactique.

3. Pansement selon la revendication 1 ou 2, **caractérisé en ce que** les aminoacides polyfonctionnels/aminoacides sont choisis parmi la glycine, la glutamine, l'alanine, l'arginine, la proline, la taurine.

4. Procédé pour la préparation d'un pansement, contenant 19 à 56% d'une ou de plusieurs protéines structurelles, choisies parmi le collagène, la gélatine, les dérivés ou les mélanges de ceux-ci, 18 à 58% d'un ou de plusieurs polysaccharides structurels, choisis parmi le chitosane, les dérivés de chitosane ou leurs mélanges, 0,5 à 10% d'acides polycarboxyliques/carboxyliques, contenant en plus d'un groupe carboxyle un ou plusieurs groupes fonctionnels, choisis parmi les groupes carboxy, hydroxy, amino, choisis parmi l'acide lactique, l'acide malique, l'acide succinique, l'acide malonique, l'acide fumarique, l'acide ascorbique, l'acide glutamique, l'acide salicylique, l'acide pyrrolidonecarboxylique ou leurs mélanges, 0,1 à 15% d'aminoacides polyfonctionnels/ aminoacides, choisis parmi l'arginine, la méthionine, la proline, la taurine, la glycine, l'alanine, la cystéine, la N-acétylcystéine ou leurs mélanges, 0 à 10% de substances actives, 0,2 à 5% de réticulant et 0 à 30% d'adjuvants et/ou d'additifs **caractérisé en ce qu'**on ajoute à une solution aqueuse du polysaccharide le(s) acide(s) polycarboxylique(s)/carboxylique(s) et à une solution aqueuse d'une protéine structurelle le(s) même(s) acide(s) polycarboxylique(s)/carboxylique(s) ou un/des acide(s) polycarboxylique(s)/carboxylique(s) différents, on dialyse ensuite les deux solutions polymères ensemble, puis on ajoute le(s) aminoacide(s) polyfonctionnel(s)/aminoacide(s) et le cas échéant des substances actives, des réticulants, des additifs et/ou des adjuvants au mélange réactionnel dialysé.

5. Procédé selon la revendication 4, **caractérisé en ce qu'**on utilise comme protéines structurelles du collagène d'origines diverses.

6. Procédé selon la revendication 5, **caractérisé en ce qu'**on utilise, comme protéines structurelles, de la gélatine de type A et de type B.

7. Procédé selon la revendication 6, **caractérisé en ce qu'**on utilise des gélatines de haut poids moléculaire présentant une valeur Bloom supérieure à 200.

8. Procédé selon l'une quelconque des revendications 4 à 7, **caractérisé en ce qu'**on utilise comme polysaccharide du chitosane, ses dérivés ou mélanges solubles dans l'eau.

9. Procédé selon l'une quelconque des revendications 4 à 8, **caractérisé en ce qu'**on utilise du chitosane présentant une masse moléculaire supérieure à 200 kDa.

10. Procédé selon l'une quelconque des revendications 4 à 9, **caractérisé en ce qu'**on utilise comme acide polycarboxylique/carboxylique l'acide malique, l'acide succinique, l'acide malonique, l'acide glutamique, l'acide pyrrolidonecarboxylique ou leurs mélanges.

11. Procédé selon la revendication 4, **caractérisé en ce que** les acides polycarboxyliques/carboxyliques sont utilisés dans un rapport 1:4 à 2:1 aux substances de haut poids moléculaire.

12. Procédé selon la revendication 4 à 11, **caractérisé en ce qu'**on mélange les solutions de polysaccharides structurels, en particulier le chitosane et les protéines structurelles, au moins 12 heures avant la dialyse.

13. Procédé selon la revendication 4 à 12, **caractérisé en ce que** la dialyse se déroule contre l'eau dans un rapport volumique de solution de polymère à eau d'au moins 1:100 pendant plus de 16 heures.

14. Procédé selon la revendication 4 à 13, **caractérisé en ce qu'**on ajoute les aminoacides polyfonctionnels/aminoacides aux solutions dialysées.

15. Procédé selon la revendication 14, **caractérisé en ce qu'**on utilise comme aminoacides l'arginine, la proline, la taurine, la glycine, l'alanine, la glutamine ou leurs mélanges.

16. Procédé selon la revendication 4, **caractérisé en ce qu'**on utilise du glutardialdéhyde comme réticulant difonctionnel.

17. Procédé selon la revendication 4 à 16, **caractérisé en ce qu'**on utilise comme substance pharmacologiquement active une superoxyde-dismutase et/ou une catalase d'origines diverses.

18. Procédé selon la revendication 17, **caractérisé en ce que** la superoxyde-dismutase et/ou la catalase est utilisée en une concentration de 0, 001 à 0,1% par rapport à la base polymère.

19. Procédé selon la revendication 4 à 17, **caractérisé en ce qu'**on utilise comme substance pharmacologiquement active du ß-carotène d'origines diverses.

20. Procédé selon la revendication 19, **caractérisé en ce qu'**on utilise comme substance pharmacologiquement active du ß-carotène sous forme liposomale.

21. Procédé selon la revendication 19 ou 20, **caractérisé en ce que** le ß-carotène est utilisé en une concentration de 0,001 à 0,05% par rapport à la base polymère.

22. Procédé selon les revendications 4 à 21, **caractérisé en ce qu'**on utilise comme adjuvants des substances antibactériennes, choisies parmi la chlorhexidine, le polyseptol, le polyhexanide, des plastifiants, des substances de haut poids moléculaire, qui assurent l'adhérence à la surface de la plaie et/ou des adjuvants qui influencent l'élimination de substances pharmaceutiquement actives.

23. Procédé selon la revendication 22, **caractérisé en ce que** les substances antibactériennes sont utilisées en une concentration de 0,01 à 0,6% par rapport à la base polymère.

24. Procédé selon la revendication 22 ou 23, **caractérisé en ce que** les additifs/adjuvants sont ajoutés au dialysat en une concentration de 10-30%.

25. Procédé selon la revendication 24, **caractérisé en ce qu'**on utilise comme adjuvants du poly(alcool vinylique) et de la polyvinylpyrrolidone.

26. Utilisation d'un pansement selon la revendication 1 à 3 ou pouvant être préparé selon la revendication 4 à 25 pour la préparation d'un agent destiné à la guérison accélérée de plaies post-traumatiques ou chirurgicales.

27. Utilisation d'un pansement selon la revendication 1 à 3 ou pouvant être préparé selon la revendication 4 à 25 pour la préparation d'un agent destiné à la guérison accélérée de brûlures au premier jusqu'au troisième degré.

28. Utilisation d'un pansement selon la revendication 1 à 3 ou pouvant être préparé selon la revendication 4 à 25 pour la préparation d'un agent destiné à la guérison accélérée de plaies infectées ou chroniques de différentes étiologies.

29. Utilisation d'un pansement selon la revendication 1 à 3 ou pouvant être préparé selon la revendication 4 à 25 pour la préparation d'un agent destiné à la guérison accélérée de plaies post-traumatiques et chirurgicales, infectées, chroniques ou de brûlures.
